(19) 

Europäisches
Patentamt
European
Patent Office
Office européen
des brevets

(11)  **EP 2 422 196 B1**

(12)  # EUROPEAN PATENT SPECIFICATION

(45) Date of publication and mention
of the grant of the patent:
**01.05.2013  Bulletin 2013/18**

(51) Int Cl.:
***G01N 33/32*** *(2006.01)*

(21) Application number: **10715584.8**

(22) Date of filing: **18.03.2010**

(86) International application number:
**PCT/IB2010/000567**

(87) International publication number:
**WO 2010/122383 (28.10.2010 Gazette 2010/43)**

(54) **PIGMENT ORIENTATION ESTIMATING METHOD**

PIGMENTORIENTIERUNGSSCHÄTZVERFAHREN

PROCÉDÉ D'ESTIMATION D'ORIENTATION DE PIGMENT

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **23.04.2009  JP 2009105202**

(43) Date of publication of application:
**29.02.2012  Bulletin 2012/09**

(73) Proprietor: **TOYOTA JIDOSHA KABUSHIKI
KAISHA
Toyota-shi,
Aichi-ken, 471-8571 (JP)**

(72) Inventors:
• **NISHIMURA, Akira
Toyota-shi, Aichi-ken, 471-8571 (JP)**
• **WATANABE, Akihiro
Toyota-shi, Aichi-ken, 471-8571 (JP)**

• **KASAI, Takashi
Tokyo 113-0033 (JP)**

(74) Representative: **Kuhnen & Wacker
Patent- und Rechtsanwaltsbüro
Prinz-Ludwig-Strasse 40A
85354 Freising (DE)**

(56) References cited:
**JP-A- 2004 152 000     JP-A- 2005 147 875
JP-A- 2006 218 425**

• **LAUDONE G M ET AL: "Modelling the shrinkage
in pigmented coatings during drying: A stick-slip
mechanism" JOURNAL OF COLLOID AND
INTERFACE SCIENCE, ACADEMIC PRESS, NEW
YORK, NY, US LNKD- DOI:10.1016/J.JCIS.
2006.08.025, vol. 304, no. 1, 1 December 2006
(2006-12-01), pages 180-190, XP024908976 ISSN:
0021-9797 [retrieved on 2006-12-01]**

**Description**

BACKGROUND OF THE INVENTION

1. Field of the Invention

The invention relates to a pigment orientation estimating method that estimates the orientation of pigment presents in a coating.

2. Description of the Related Art

In order to ensure stable coating quality, there is a need for understanding how a coating appearance varies when the characteristic of a paint and/or a painting condition are changed. Within the coating appearance, the orientation of pigments in the coating is the most important.

LAUDONE G M ET AL: "Modelling the shrinkage in pigmented coatings during drying: A stick-slip mechanism", JOURNAL OF COLLOID AND INTERFACE SCIENCE, ACADEMIC PRESS, NEW YORK, NY, US LNKD- DOI: 10.1016/J.JCIS.2006.08.025, vol. 304, no. 1, 1 December 2006 (2006-12-01), pages 180-190. ISSN: 0021-9797 discloses a method of modelling the shrinkage in pigmented coatings during drying. The method generates a simulation of the void space of a porous sample, whereby software generates spherical *"skeletal elements"* which are representative of the solid phase. The algorithm also simulates the skeletal element size distributions of paper coating layers, by generating spherical elements between cubic pores.

Japanese Patent Application Publication No. 2005-147875 (JP-A-2005-147875) describes a method of evaluating the orientation of pigments. In the method, a plurality of painted plates that are painted under different paint conditions and/or different painting conditions are prepared. Then, the positional information of pigments in the coating is acquired from a cut piece of each painted plate, and the inclinations of pigments to a coating surface are calculated from the positional information.

Japanese Patent Application Publication No. 2006-218425 (JP-A-2006-218425) describes a method of estimating a coating result. In the method, a plurality of painted test panels are prepared, and the visual characteristic value of each test panel is measured and stored in a database. Then, the database is used and subjected to multiple regression analysis.

In addition, Japanese Patent Application Publication No. 2004-152000 (JP-A-2004-152000) describes a technique of an image simulation method for a painted appearance. In the image simulation method, the arrangement of coloring materials in a coating is input to calculate the colored state of a virtually painted object, and the calculated colored state of the virtually painted object is displayed in image.

In the case of the technique described in JP-A-2005-147875, there are many combinations of paint conditions and painting conditions, and, in addition, it takes time to adjust the evaluation condition, adjust the temperature and humidity in a painting booth or adjust a paint. Thus, a method of evaluating the appearance by preparing a painted plate one by one requires much expense in time and effort, resulting in an increase in cost.

When the number of pieces evaluated is limited as in the case of the technique described in JP-A-2006-218425, there is a problem that it is difficult to detect a malfunction under an unexpected environmental condition. When the number of pieces evaluated is increased, there is a problem that it is necessary to take much time for evaluation.

Then, the technique described in JP-A-2004-152000 is to input the specified arrangement of coloring materials (the orientation of pigments), but the technique is not to estimate the orientation of pigments. As described above, the process of drying a coating is important to the orientation of pigments; however, there is no method for estimating the orientation of pigments in consideration of the drying process.

SUMMARY OF THE INVENTION

The invention provides a pigment orientation estimating method according to claim 1 that is able to accurately and easily estimate the orientation of pigments in a coating depending on the characteristic of a paint and/or a painting condition.

The invention relates to a pigment orientation estimating method that estimates the orientation of pigments in a coating. The method includes: setting the orientation of the pigments when a paint is applied; and calculating the orientation of the pigments at time, at which a predetermined period of time has elapsed from when the paint is applied, on the basis of a shrinkage rate gradient of the paint in a thickness direction of the coating in the process of drying the paint.

The orientation of the pigments when a paint is applied is set, and the orientation of the pigments at time at which a predetermined period of time has elapsed from when the paint is applied is calculated on the basis of a shrinkage rate gradient of the paint in a thickness direction of the coating in the process of drying the paint. Thus, for example, by assuming the state where the pigments are dispersed in the paint as the state where spheroids are present in viscous fluid, and by applying the motion model in which the spheroids present in the fluid rotate by a difference in velocity of flow of the fluid, it is possible to accurately and easily estimate the orientation of the pigments depending on the characteristic of a paint and a painting condition.

The method may further include: a first step of setting the characteristic of a paint and a painting condition; a second step of setting the orientation of the pigments when the paint is applied and setting zero for time at which the paint is applied; a third step of advancing the time by one unit time; and a fourth step of calculating the orientation of the pigments at the time that is advanced by one unit time on the basis of a shrinkage rate gradient of the paint in a thickness direction of the coating in the process of drying the paint.

With the above aspect of the invention, the characteristic of a paint and a painting condition are set, the orientation of the pigments when a paint is applied is set, and the orientation of the pigments at time that is advanced by one unit time is calculated on the basis of a shrinkage rate gradient of the paint in a thickness direction of the coating in the process of drying the paint. Thus, for example, by assuming the state where the pigments are dispersed in the paint as the state where spheroids are present in viscous fluid, and by applying the motion model in which the spheroids present in the fluid rotate by a difference in velocity of flow of the fluid, it is possible to accurately and easily estimate the orientation of pigments at the time that is advanced by one unit time and depending on the characteristic of a paint and a painting condition.

The pigment orientation estimating method may further include a fifth step of determining whether the coating has been dried, subsequent to the fourth step, wherein a process may return to the third step when it is determined in the fifth step that the coating has not been dried, and the third step and the fourth step may be repeated until it is determined in the fifth step that the coating has been dried.

By so doing, it is possible to calculate the orientation of the pigments in the dried coating. Particularly, the third step and the fourth step are repeated until it is determined in the fifth step that the coating has been dried, so the orientation of the pigments may be calculated in consideration of a situation, such as the viscosity of the paint and the positions of the pigments, that varies momentarily from when the paint is applied to when the coating has been dried. Therefore, it is possible to accurately estimate the orientation of the pigments in the dried coating.

The shrinkage rate gradient of the coating may be calculated on the basis of the characteristic of the paint and a painting condition.

Then, the orientation of the pigments may be calculated using a mathematical expression that determines the rotating state of a spheroid in viscous fluid.

By so doing, the orientation of the pigments is calculated using a mathematical expression that determines the rotating state of a spheroid in viscous fluid. Thus, it is possible to calculate a variation in rotation angle of each pigment depending on a shrinkage of the coating in the thickness direction of the coating. Hence, it is possible to accurately and easily estimate the orientation of the pigments.

According to the invention, by assuming the state where the pigments are dispersed in the paint as the state where spheroids are present in viscous fluid, and by applying the motion model in which the spheroids present in the fluid rotate by a difference in velocity of flow of the fluid, it is possible to accurately and easily estimate the orientation of the pigments depending on the characteristic of a paint and a painting condition.

BRIEF DESCRIPTION OF THE DRAWINGS

The foregoing and further objects, features and advantages of the invention will become apparent from the following description of example embodiments with reference to the accompanying drawings, wherein like numerals are used to represent like elements and wherein:

FIG. 1 is a flowchart that illustrates a pigment orientation estimating method according to an embodiment of the invention;
FIG. 2A and FIG. 2B are views that illustrate a change of orientation of pigments due to drying shrinkage of a coating;
FIG. 3 is a vertical cross-sectional view that illustrates force acting on a spheroid in viscous fluid;
FIG. 4 is a view that illustrates the shrinkage rate gradient of a coating in the thickness direction; and
FIG. 5 is a graph that shows the data of pigment orientation when a paint is applied and the data of pigment orientation after the coating is dried, estimated through the pigment orientation estimating method.

DETAILED DESCRIPTION OF EMBODIMENTS

As described above, there is a need for understanding how a coating appearance varies when the characteristic of a paint and/or a painting condition are changed. Then, within the coating appearance, the orientation of pigments is the most important.

For example, in the case of aluminum pigments, the more the orientation of the pigments is aligned parallel to a painted surface, the better the appearance of metallic color is. Therefore, by estimating the orientation of pigments on a computer, it is possible to omit the time and effort for testing the appearance of an actually prepared painted plate. The pigment orientation estimating method according to the present embodiment is implemented by executing a program for estimating the orientation of pigments on a computer.

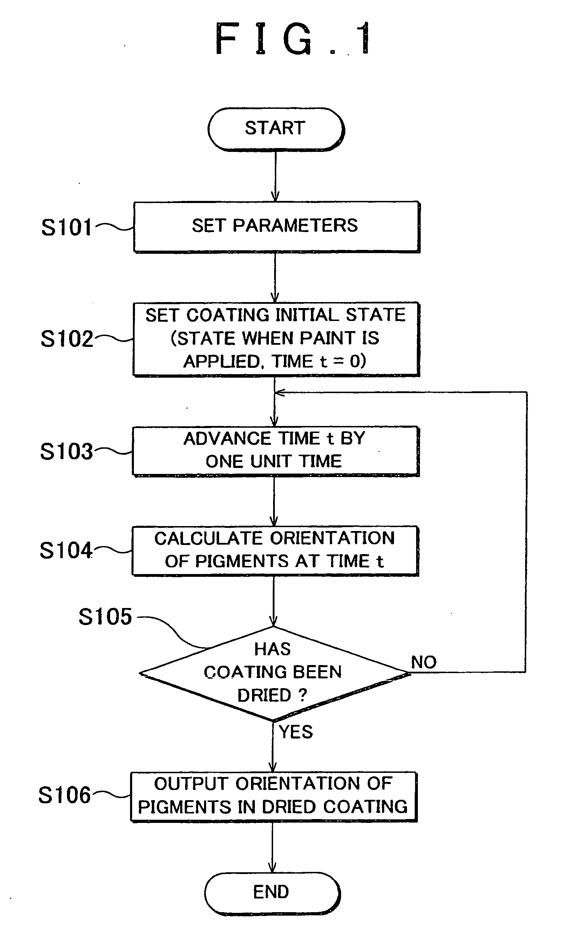

FIG. 1 is a flowchart that illustrates a pigment orientation estimating method according to an embodiment of the invention. First, in step S101, parameters of the characteristic of a paint, a painting condition, and the like, are set (first step). Then, in step S102, a state at the time when a paint is applied (coating initial state) is set, and the time t at which a paint is applied is set at zero (t = 0) (second step).

Table 1 is a listing of an example of parameters, and the like, set in step S101 and step S102. For example, (1) reflective material size range, (2) reflective material size ($\mu$m), (5) the amount of paint per unit area, (8) solvent specific gravity, (11) reflective material mean diameter ($\mu$m), and the like, are set as parameters. Then, (3) orientation angle range (from 1), (4) orientation angles (deg) of pigments, and the like, indicating the orientation of pigments when a paint is applied are set as the paint initial state.

Table 1

| | Input Items | Associated Functions | | | | |
|---|---|---|---|---|---|---|
| | | Coating Shrinkage | Reflective Material High Density | Leveling | Incomplete Leveling | Presence or Absence of Convection |
| 0 | Version | | | | | |
| 1 | Reflective Material Size Range | O | O | O | O | |
| 2 | Reflective Material Size ($\mu$m), Indicating Size Section Range Size Range + 1 | O | O | O | O | |
| 3 | Orientation Angle Range (From 1) | O | O | O | O | |
| 4 | Orientation Angles (deg), Angle Section Range | O | O | O | O | |
| 5 | Paint Amount (Weight Of Paint Used) Per Unit Area (g/m$^2$) | O | O | O | O | O |
| 6 | Paint Weight Residual Percentage (NV to 1) in Spraying Process 1 When No Volatilization | O | O | O | O | O |
| 7 | Solid Content NV (0 to 1) | O | O | O | O | O |
| 8 | Solvent Specific Gravity (Density of Solvent 100%) (kg/m$^3$) | O | O | O | O | O |

(continued)

|  | Input Items | Associated Functions | | | | |
|---|---|---|---|---|---|---|
|  |  | Coating Shrinkage | Reflective Material High Density | Leveling | Incomplete Leveling | Presence or Absence of Convection |
| 9 | Dried Thickness (μm) 13 μm | O | O | O | O | O |
| 10 | Time Required for Drying (s) | O | O | O | O |  |
| 11 | Aluminum Foil NJ Reflective Material Mean Diameter (μm) About 14 μm (up to This Item Indispensable) | O | O | O | O |  |
| 12 | Reflective Material Diameter Distribution Logarithmic Normal Distribution (Currently Not Used) Effective Flag of Shrinkage of Coating (Density of Reflective Material Is High) |  | O |  |  |  |
| 13 | (Following Three Items Are Required When Density of Reflective Material Is High) Weight Ratio of Reflective Material to All Solid Content (PWC) |  | O |  |  |  |
| 14 | (0 to 1) (Required When Reflective Material Density Is Derived As Well) Reflective Material Thickness μm About 0.5 μm | Δ | O |  |  |  |

(continued)

|  | Input Items | Associated Functions | | | | |
|---|---|---|---|---|---|---|
|  |  | Coating Shrinkage | Reflective Material High Density | Leveling | Incomplete Leveling | Presence or Absence of Convection |
| 15 | (Required for Reflective Material High Density Calculation, Optionally for Shrinkage of Coating) Wave Amplitude during Painting, Ratio to Mean Thickness (0 to 1) |  |  | O | O |  |
| 16 | (This Item > 0 When There Is Leveling) |  |  |  | O |  |
| 17 | Effective Flag of Incomplete Leveling |  |  |  | O |  |
| 18 | Wave Period during Painting (mm) |  |  |  | O |  |
| 19 | Paint Surface Tension (N/m) |  |  |  | O |  |
| 20 | Time Range (Viscosity Sampling) |  |  |  | O | O |
| 21 | Time (s), Starting from 0 (Viscosity Sampling) |  |  |  | O |  |
| 22 | Viscosity (Pa●s), Value at Low Shear Rate |  |  |  | O | O |
| 23 | Effective Flag of Presence or Absence of Convection |  |  |  |  | O |
| 24 | Thermal Expansion Coefficient of Paint |  |  |  |  | O |
| 25 | Thermal Conductivity (W/m/K) of Paint |  |  |  |  | O |

(continued)

| | Input Items | Associated Functions | | | | |
|---|---|---|---|---|---|---|
| | | Coating Shrinkage | Reflective Material High Density | Leveling | Incomplete Leveling | Presence or Absence of Convection |
| 26 | Specific Heat Capacity at Constant Pressure (J/kg/K) of Paint | | | | | O |
| 27 | Baking Temperature - Room Temperature (K) | | | | | O |

Subsequently, in step S103, the time t is advanced by one unit time (third step). In the present embodiment, one unit time is set to one second. Then, in step S104, the orientation of pigments at the time that is advanced by one unit time is calculated on the basis of the shrinkage rate in the thickness direction in the process of drying a paint (fourth step). Here, the time evolution differential equation of the pigment orientation, which will be described later, is used to calculate the orientation of pigments.

Then, in step S105, it is determined whether the coating has been dried (fifth step). In the present embodiment, the above determination is made on the basis of a period of time elapsed. When a predetermined period of time has elapsed from application of a paint (YES in step S 1 05), it is determined that the coating has been dried.

On the other hand, when the predetermined period of time has not elapsed from application of a paint (NO in step S105), it is determined that the coating has not been dried yet, and the process returns to step S103 again. After that, until it is determined in step S105 that the coating has been dried, the process in step S103 and the process in step S 104 are repeatedly executed.

Then, when it is determined in step S105 that the coating has been dried, the process proceeds to step S106. In step S 106, the orientation of pigments after the coating has been dried is output.

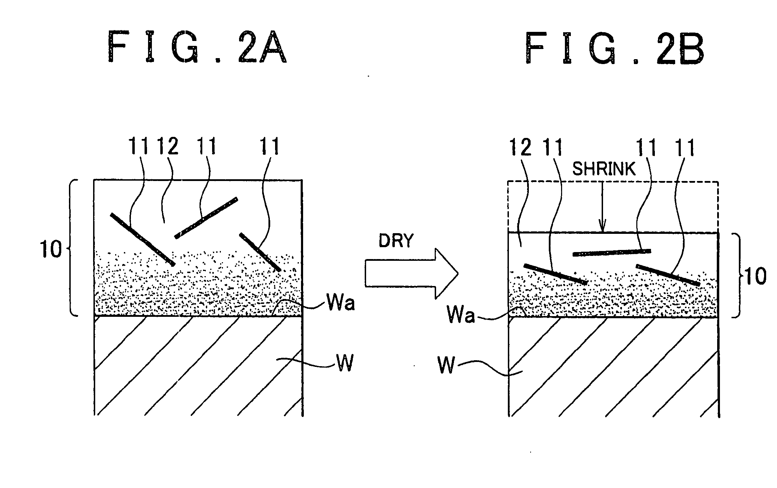

Next, the processes in the pigment orientation estimating method will be described in detail. FIG 2A and FIG. 2B are schematic views that illustrate a change of orientation of pigments due to drying shrinkage of a coating. FIG 2A is a cross-sectional view that shows the state of a paint immediately after application of a paint. FIG. 2B is a cross-sectional view that shows the state of the dried coating.

In the process of drying a paint (curing process), as a solvent included in a paint 12 evaporates from the state shown in FIG. 2A, a coating 10 shrinks, and the thickness of the coating 10 reduces. Then, with a reduction in the thickness of the coating 10, the orientation of pigments 11 dispersed in the paint varies, and, as shown in FIG 2B, the orientation of the pigments 11 varies so as to be parallel to a painted surface Wa of a painted object W.

Immediately after application of the paint 12, as shown in FIG. 2A, the pigments 11 are dispersed in the paint 12. If this is assumed that spheroids are present in viscous fluid, a variation in the orientation of the pigments 11 due to a reduction in the thickness of the coating 10 as shown in FIG. 2A and FIG. 2B may be modeled as the rotational motion of the spheroid 11 due to a difference in velocity of flow of fluid 12, exerted on portions of the pigments 11. Note that the spheroid is a solid made by rotating an ellipse, and may be classified into an oblate spheroid, a spherical spheroid, and a prolate spheroid.

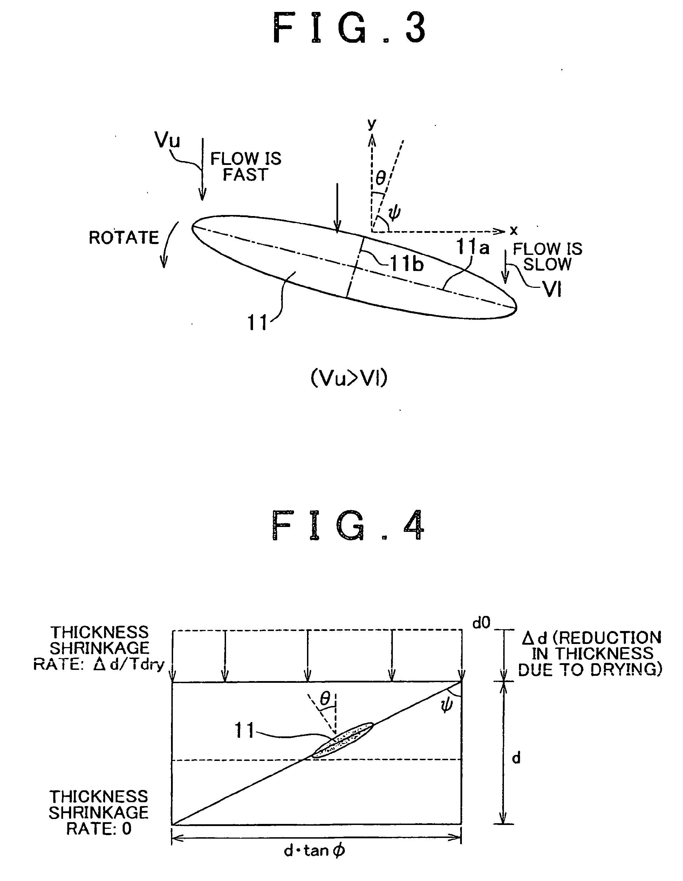

FIG. 3 is a vertical cross-sectional view that illustrates force acting on the spheroid with a reduction in the thickness of the coating. The spheroid 11 has a major axis 11a and a minor axis 11b. It is assumed that the spheroid 11 is oriented in position such that the inclination angle of the minor axis 11b with respect to a direction parallel to the painted surface (x-axis direction in the drawing) is $\phi$ and the inclination angle of the minor axis 11b with respect to a direction perpendicular to the painted surface (y-axis direction in the drawing) is $\theta$.

Then, with a reduction in the thickness of the coating, flow occurs in the paint 12 from an upper layer side in the thickness direction, located adjacent to the coating surface, toward a lower layer side in the thickness direction, located adjacent to the painted surface (in FIG. 3, from the upper side toward the lower side). Within the flow in the thickness direction in the paint 12, flow Vu at the upper layer in the thickness direction is faster than flow Vl at the lower layer in the thickness direction (Vu > Vl), and force acts on the spheroid 11 to rotate the spheroid 11 along the painted surface Wa. Thus, the spheroid 11 rotates toward a direction in which the spheroid 11 is parallel to the painted surface Wa.

According to Jeffery's theoretical formula, the rotational motion of the spheroid in fluid may be expressed by the mathematical expression (1).

$$\frac{d\varphi}{dt} = -\frac{\dot{\gamma}}{2}\left(1 + \frac{r^2 - 1}{r^2 + 1}\left(\sin^2\varphi - \cos^2\varphi\right)\right) \qquad \cdots (1)$$

where $\phi$ is an angle made between the direction of the axis of the spheroid and the direction along the painted surface, r is an aspect ratio (major axis / minor axis) of the spheroid (in the case of an oblate spheroid, the spheroid becomes a complete disk at the limit of the major axis >> the minor axis), and

$\gamma$ is a shear rate (shrinkage rate gradient).

Here, the shear rate $\gamma$ (shrinkage rate gradient) may be obtained under the following concept.

FIG. 4 is a view that illustrates the shrinkage rate gradient of a coating in the thickness direction. Where a shrinkage of the coating 10 in the thickness direction is $\Delta$d and a period of time until the coating 10 is cured is Tdry, the shrinkage rate in the thickness direction is $\Delta$d/Tdry if it is regarded as invariable. Then, the shrinkage rate gradient is $\Delta$d/Tdry/(d(t)tan$\phi$) as shown in FIG 4. Here, the thickness d(t) is expressed by d(t) = d0 - $\Delta$d/Tdry$\times$t.

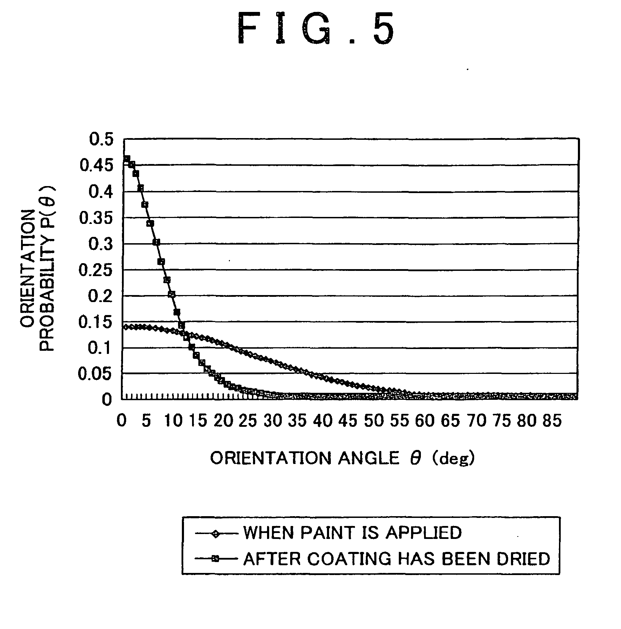

The pigment orientation is a probability P($\theta$) of occurrence of an orientation angle $\theta$, and is expressed by Gaussian distribution (for example, see FIG. 5). A variation over time in the probability P($\theta$) of the pigment orientation is expressed by the time evolution differential equation shown in the following mathematical expression (2).

$$\frac{dP}{dt} = \frac{\partial P}{\partial \theta}\frac{\partial \theta}{\partial t} + P\frac{\partial \dot{\theta}}{\partial \theta} \qquad \cdots (2)$$

Note that $\theta$ indicates a state where the pigment 11 is laid parallel to the painted surface Wa when the angle made between the minor axis 11b direction of the spheroid 11 and the direction perpendicular to the painted surface Wa (y-axis direction). The left-hand side of the above mathematical expression (2) indicates a variation in the probability P per unit time, and the second term in the right-hand side is a term for maintaining the area of Gaussian distribution at constant. As Gaussian distribution expands or contracts laterally, the height of distribution varies.

Then, the first term $\partial\theta/\partial t$ in the right-hand side indicates a variation in scale $\theta$ of the probability P($\theta$), and is obtained by reversing the sign of a variation over time of the orientation angle $\theta$ calculated by Jeffery's equation. When the time evolution differential equation of the pigment orientation distribution, shown in the mathematical expression (2), is solved, the pigment orientation after the coating has been dried is obtained. In the present embodiment, the above differential equation is solved by Runge-Kutta method.

FIG 5 is a graph that shows the data of pigment orientation when the paint is applied and the data of pigment orientation after the coating is dried, estimated through the pigment orientation estimating method. The thickness d of the dried coating 10 is set at about one third of the thickness at the time when the paint is applied (43 $\mu$m $\rightarrow$ 13 $\mu$m). According to the results of calculation, the half-width of the orientation distribution changes from 30 degrees at the time when the paint is applied into 9 degrees after the coating has been dried, so the orientation is improved.

With the above described pigment orientation estimating method, the characteristic of the paint 12 and the painting condition are set, the orientation of the pigments 11 when the paint 12 is applied is set, and the orientation of the pigments at time t that is advanced by one unit time is calculated on the basis of the shrinkage rate gradient in the thickness direction in the process of drying the paint 12. Thus, by assuming the state where the pigments 11 are dispersed in the paint 12 as the state where spheroids are present in viscous fluid, and applying the motion model in which the spheroids present in the fluid rotate by a difference in velocity of flow of the fluid, it is possible to accurately and easily estimate the orientation of the pigments 11 at time that is advanced by one unit time and depending on the characteristic of the paint 12 and the painting condition.

Then, the processes in step S103 and step S104 are repeated until it is determined in step S105 that the coating 10 has been dried, so the orientation of the pigments 11 may be calculated in consideration of a situation, such as the viscosity of the paint 12 and the positions of the pigments 11, that varies momentarily from when the paint is applied to when the coating has been dried. Therefore, it is possible to accurately estimate the orientation of the pigments 11 in the dried coating.

In the above described embodiment, the pigment orientation estimating method is implemented by a computer program, for example; however, the pigment orientation estimating method is not limited to this configuration. For example, the pigment orientation estimating method may be implemented by a combination of software and hardware or may be implemented by a device.

In the above described embodiment, calculation is made from when the paint is applied to when the coating has been dried, for example; however, a period of time during which calculation is made is not limited to the period of time from when the paint is applied to when the coating has been dried. Instead, the period of time may be part of that period of time.

In addition, in the above described embodiment, the orientation of pigments is calculated every unit time, for example. Instead, as long as a period of time, or the like, during which, for example, the viscosity, or the like, of a coating varies proportionally to an elapsed time from when a paint is applied, the orientation of pigments at time at which a predetermined period of time has elapsed from when the paint is applied may be calculated.

**Claims**

1. A pigment orientation estimating method that estimates the orientation of pigments (11) in a coating (10), comprising the steps of:

    (a) setting the orientation of the pigments (11) at the time of applying a paint (12); and
    (b) calculating the orientation of the pigments (11) at time (t), at which a predetermined period of time (t) has elapsed from when the paint is applied, on the basis of a shrinkage rate gradient of the paint (12) in a thickness direction of the coating (10) in the process of drying the paint (12);

    wherein the pigment orientation is a probability $(P((\Theta))$ of occurrence of an orientation angle $(\Theta)$ and is expressed by Gaussian distribution;
    wherein a variation over time (t) in the probability $(P(\Theta))$ of the pigment orientation is expressed by a time evolution differential equation $(dP(\Theta)/dt))$ which when solved yields the pigment orientation after the coating (10) has been dried; and
    wherein pigment orientation is estimated by the use of a computer implemented fluid motion model.

2. The pigment orientation estimating method according to claim 1, further comprising the steps of:

    (c) before step (a), setting the characteristic of the paint (12) and a painting condition; and
    (d) after step (a) and before step (b), advancing the time (t) by one unit time;

    wherein in step (a) the time (t) at which the paint (12) is applied is set to zero, and in step (b) the predetermined period of time (t) is one unit time.

3. The pigment orientation estimating method according to claim 2, further comprising the step of:

    (e) after step (b), determining whether the coating (10) has been dried,

    wherein a process returns to step (d) when it is determined in step (e) that the coating (10) has not been dried, and step (d) and step (b) are repeated until it is determined in step (e) that the coating (10) has been dried.

4. The pigment orientation estimating method according to any one of claims 1 to 3, wherein the shrinkage rate gradient is calculated on the basis of the characteristic of the paint (12) and a painting condition.

5. The pigment orientation estimating method according to any one of claims 1 to 4, wherein the orientation of the pigments (11) is calculated using a mathematical expression that determines the rotating state of a spheroid in viscous fluid.

**Patentansprüche**

1. Verfahren zur Schätzung einer Pigmentorientierung, das die Orientierung von Pigmenten (11) in einer Beschichtung (10) schätzt, mit den Schritten:

    (a) Einstellen der Orientierung der Pigmente (11) zum Zeitpunkt des Auftragens einer Farbe (12); und
    (b) Berechnen der Orientierung der Pigmente (11) zu einer Zeit (t), bei der eine vorbestimmte Zeitspanne (t) seit dem Auftragen der Farbe verstrichen ist, auf der Basis eines Schrumpfraten-Gradienten der Farbe (12) in einer Dickenrichtung der Beschichtung (10) beim Vorgang des Trocknens der Farbe (12);

    wobei die Pigmentorientierung eine Wahrscheinlichkeit $(P(\Theta))$ des Auftretens eines Orientierungswinkels $(\Theta)$ ist und

durch eine Gauß-Verteilung ausgedrückt ist;

wobei eine Änderung der Wahrscheinlichkeit (P(Θ)) der Pigmentorientierung über die Zeit (t) durch eine zeitabhängige Differenzialgleichung (dP(Θ)/dt) ausgedrückt ist, welche, wenn sie gelöst wird, die Pigmentorientierung nach dem Trocknen der Beschichtung (10) liefert; und

wobei die Pigmentorientierung unter Verwendung eines computerimplementierten Fluidbewegungs-Modells geschätzt wird.

**2.** Verfahren zur Schätzung einer Pigmentorientierung nach Anspruch 1, das ferner die nachfolgenden Schritte aufweist:

(c) vor Schritt (a), Einstellen der Eigenschaft der Farbe (12) und einer Auftragungsbedingung; und
(d) nach Schritt (a) und vor Schritt (b), Erhöhen der Zeit (t) um eine Einheitszeit;

wobei in Schritt (a) die Zeit (t), bei der die Farbe (12) aufgetragen wird, auf Null gesetzt wird, und in Schritt (b) die vorbestimmte Zeitspanne (t) eine Einheitszeit ist.

**3.** Verfahren zur Schätzung einer Pigmentorientierung nach Anspruch 2, ferner den Schritt aufweisend:

(e) nach Schritt (b), Ermitteln, ob die Beschichtung (10) getrocknet ist,

wobei ein Vorgang zu Schritt (d) zurückkehrt, wenn in Schritt (e) bestimmt wird, dass die Beschichtung (10) nicht getrocknet ist, und
Schritt (d) und Schritt (b) werden wiederholt, bis in Schritt (e) bestimmt wird, dass die Beschichtung (10) getrocknet worden ist.

**4.** Verfahren zur Schätzung einer Pigmentorientierung nach einem der Ansprüche 1 bis 3, wobei der Schrumpfraten-Gradient auf Basis der Eigenschaft der Farbe (12) und einer Auftragungsbedingung berechnet wird.

**5.** Verfahren zur Schätzung einer Pigmentorientierung gemäß einem der Ansprüche 1 bis 4, wobei die Orientierung der Pigmente (11) unter Verwendung eines mathematischen Ausdrucks berechnet wird, welcher den Rotationszustand eines Sphäroids in einem viskosen Fluid bestimmt.

**Revendications**

**1.** Procédé d'estimation d'orientation de pigments qui estime l'orientation de pigments (11) dans un revêtement (10), comprenant les étapes consistant :

(a) à fixer l'orientation des pigments (11) au moment de l'application d'une peinture (12) ; et
(b) à calculer l'orientation des pigments (11) au temps (t), auquel une période prédéterminée de temps (t) s'est écoulée depuis le moment où la peinture a été appliquée, sur la base d'un gradient de vitesse de contraction de la peinture (12) dans le sens de l'épaisseur du revêtement (10) dans le processus de séchage de la peinture (12) ;

dans lequel l'orientation de pigments est une probabilité (P(Θ)) d'occurrence d'un angle (Θ) d'orientation et est exprimée par une distribution gaussienne,
dans lequel la variation dans le temps (t) de la probabilité (P(Θ)) de l'orientation de pigments est exprimée par une équation différentielle d'évolution dans le temps (dP(Θ)/dt) qui lorsqu'elle est résolue donne l'orientation de pigments après que le revêtement (10) a séché, et
dans lequel l'orientation de pigments est estimée par l'utilisation d'un modèle de mouvement de fluide mis en oeuvre en ordinateur.

**2.** Procédé d'estimation d'orientation de pigments selon la revendication 1, comprenant en outre les étapes consistant :

(c) avant l'étape (a), à fixer la caractéristique de la peinture (12) et une condition de l'opération de peinture ; et
(d) après l'étape (a) et avant l'étape (b), à faire avancer le temps (t) d'une unité de temps,

dans lequel, à l'étape (a), le temps (t) auquel la peinture (12) est appliquée est fixé à zéro, et à l'étape (b), la période prédéterminée de temps (t) est une unité de temps.

3. Procédé d'estimation d'orientation de pigments selon la revendication 2, comprenant en outre l'étape consistant :

(e) après l'étape (b), à déterminer si le revêtement (10) est sec,

dans lequel le processus retourne à l'étape (d) lorsqu'il est déterminé à l'étape (e) que le revêtement (10) n'est pas sec, dans lequel on répète l'étape (d) et l'étape (b) jusqu'à ce qu'il soit déterminé à l'étape (e) que le revêtement (10) est sec.

4. Procédé d'estimation d'orientation de pigments selon l'une quelconque des revendications 1 à 3, dans lequel le gradient de vitesse de contraction se calcule sur la base de la caractéristique de la peinture (12) et d'une condition de l'opération de peinture.

5. Procédé d'estimation d'orientation de pigments selon l'une quelconque des revendications 1 à 4, dans lequel l'orientation des pigments (11) se calcule en utilisant une expression mathématique qui détermine l'état de rotation d'un sphéroïde dans un fluide visqueux.

# FIG.1

```
        ┌─────────────┐
        │    START    │
        └──────┬──────┘
               │
               ▼
S101 ──┌─────────────────┐
       │  SET PARAMETERS  │
       └────────┬─────────┘
                │
                ▼
S102 ──┌────────────────────────┐
       │ SET COATING INITIAL STATE │
       │ (STATE WHEN PAINT IS     │
       │ APPLIED, TIME t = 0)     │
       └────────┬───────────────┘
                │  ◄──────────────────┐
                ▼                      │
S103 ──┌────────────────────┐         │
       │  ADVANCE TIME t BY   │         │
       │  ONE UNIT TIME       │         │
       └────────┬───────────┘         │
                │                      │
                ▼                      │
S104 ──┌──────────────────────┐       │
       │ CALCULATE ORIENTATION │       │
       │ OF PIGMENTS AT TIME t │       │
       └────────┬────────────┘       │
                │                      │
                ▼                      │
S105        ◇ HAS COATING       NO     │
             BEEN DRIED ? ──────────────┘
                │ YES
                ▼
S106 ──┌────────────────────────┐
       │ OUTPUT ORIENTATION OF   │
       │ PIGMENTS IN DRIED COATING│
       └────────┬───────────────┘
                │
                ▼
        ┌─────────────┐
        │     END     │
        └─────────────┘
```

# FIG.2A

# FIG.2B

EP 2 422 196 B1

# FIG.3

(Vu>Vl)

# FIG.4

# F I G . 5

Legend:
- ◇ WHEN PAINT IS APPLIED
- ▨ AFTER COATING HAS BEEN DRIED

Y-axis: ORIENTATION PROBABILITY P($\theta$)

X-axis: ORIENTATION ANGLE $\theta$ (deg)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005147875 A
- JP 2006218425 A
- JP 2004152000 A

**Non-patent literature cited in the description**

- Modelling the shrinkage in pigmented coatings during drying: A stick-slip mechanism. **LAUDONE G M et al.** JOURNAL OF COLLOID AND INTERFACE SCIENCE. ACADEMIC PRESS, 01 December 2006, vol. 304, 180-190